# EUROPEAN PATENT APPLICATION

(11) **EP 1 696 034 A1**
(43) Date of publication of application: **30.08.2006**
(21) Application number: 04807517.0
(22) Date of filing: 15.12.2004
(51) Int. Cl.: C12N 15/63

(54) **NOVEL METHOD OF NUCLEIC ACID TRANSFER**

(30) Priority: 19.12.2003 JP 2003423004
(71) Applicant: Dainippon Sumitomo Pharma Co., Ltd., Osaka 541-8524 (JP); KOKEN CO., LTD., Toshima-ku, Tokyo 171-0031 (JP)
(72) Inventor: MINAKUCHI, Yoshiko Dainippon Sumimoto Phar.Co.,Ltd, Ibaraki-shi, Osaka 567-0878 (JP); OCHIYA, Takahiro, Tokyo 1040045 (JP)
(74) Representative: Vossius & Partner
(86) International application number: PCT/JP2004/019160
(87) International publication number: WO 2005/061717

(57) **Abstract**

The present invention provides a method of nucleic acid transfer comprising the following steps (a) and (b):
(a) contacting a nucleic acid with a cell in a medium; and
(b) following the step (a), contacting the medium of (a) with a high-concentration solution of a metal salt,
a nucleic acid transfer agent comprising solid metal salt or a high-concentration solution of a metal salt as an ingredient, and the like.

## Description

### TECHNICAL FIELD

The present invention relates to a novel method of nucleic acid transfer, and specifically to a novel method of transferring a nucleic acid using a solution containing a metal salt at a high concentration.

### BACKGROUND ART

With the progress of genomic science, many disease-related genes have been identified, and the functional analysis thereof is of urgent need. However, it is difficult to elucidate the function of a gene in cultured cells directly by conventional techniques which are currently used broadly for analyzing gene function, such as transcriptome analysis that uses the DNA microarray technique or yeast two-hybrid assay. Therefore, establishment of a method for analyzing gene function exhaustively at cell or individual level whereby the gene function analysis can be directly connected to the identification of a therapeutic target molecule and the drug invention has been needed. The functional analysis of a gene at cell and individual level is conducted by a technique comprising causing overexpression or knock down of mRNA encoded by a gene to be evaluated with the use of a nucleic acid prepared on the basis of sequence of said gene in a cell, and analyzing the consequential functional changes of the cell. Recently, it has become available a technique whereby the gene function analysis based on various parameters can be performed at cell level using the multiimage analyzer, and therefore establishment of a method for regulating gene expression is a critical issue to perform the functional analysis of genes.

As a nucleic acid that regulates gene expression, there are plasmid DNAs (pDNAs) and virus vectors for gene overexpression, and antisense DNAs (AS-DNAs) and short interfering RNAs (siRNAs) for gene knockdown. A nucleic acid and cell membrane are both anionic, and therefore it is hard to introduce the nucleic acid itself into a cell directly because of the electric repulsion. Accordingly, when performing the gene function analysis using cells, it is necessary to use a conventional technique for introducing a nucleic acid into cells which has been developed so far, such as virus vector, electroporation, calcium phosphate coprecipitation, DEAE-dextran, lipofection, or polymer micelle vector technique.

According to the virus vector method (Mah C et al, Virus-based gene delivery systems., *Clin Pharmacokinet.,* 2002;41(12):901-911), an intended gene can be transferred into cells and expressed in high yield just by inserting the gene into a virus vector and adding the resulting vectors to the cells by virtue of infectious capacity of virus vectors. However, this technique has drawbacks. That is, since a nucleic acid is transferred into a cell through infection, the cell may develop a defense mechanism against infection, which possibly arises a noise(s) affecting the detection of functions peculiar to an intended gene. Further, this technique has a limitation on the length of a gene to be inserted into a virus vector, and requires complicated procedures for introducing a gene, amplifying and purifying viruses, and hence is not suited to exhaustive functional analysis applicable to many kinds of genes.

When transferring pDNA, AS-DNA or siRNA into cells, it is necessary to use the above-mentioned technique such as the electroporation technique (Gehl J, Electroporation: theory and methods, perspectives for drug delivery, gene therapy and research., *Acta Phisiol scand.,* 2003; 117(4): 437-47), the calcium phosphate coprecipitation technique (Batard P et al, Transfer of high copy number plasmid into mammalian cells by calcium phosphate transfection., *Gene,* 2001; 270:61-68), the DEAE-dextran technique (Holter W et al, Efficient gene transfer by sequential treatment of mammalian cells with DEAE-dextran and deoxyribonucleic acid., *Exp Cell Res.* 1989;184(2): 546-551), the lipofection technique (Rocha A et al, Improvement of DNA transfection with cationic liposomes., L *Physiol Biochem.* 2002; 58(1): 45-56), and the technique that makes use of a high molecule polymer (De Smedt SC, et al, Cationic polymer Based Gene Delivery Systems., *Pharm. Res.* 2000; 17(2): 113-26).

According to the electroporation technique, a nucleic acid can be transferred into a cell by suspending cells into a nucleic acid solution and pulsing the suspension with high direct-current voltage to increase the cell membrane permeability. Although this technique can give high-yield nucleic acid transfer, it may bring about significant damage of cells. Therefore, this technique is not suited to a purpose of analyzing functional change of cells after nucleic acid transfer.

The calcium phosphate coprecipitation technique transfers a nucleic acid into a cell by virtue of endocytosis. This technique is rather poor in reproducibility and transfer efficiency, and, therefore, is inapplicable to the gene function analysis that requires stable gene transfer.

According to the DEAE-dextran technique, the lipofection technique, and the technique that makes use of a high-molecule polymer, a nucleic acid is transferred into a cell by virtue of fusion with the cell membrane. Among these techniques, the lipofection technique is superior to others in view of transfer efficiency, simplicity of use, versatility and reproducibility. However, the lipofection technique is known to be highly cytotoxic, which can be problematic in the gene function analysis where the cell viability is significant. Further, it is necessary to use a complex prepared by mixing nucleic acids and liposome reagents to transfer a gene into cells by the lipofection technique. Notwithstanding the high reproducibility, even a subtle nuance arisen from the preparation conditions of complexes may affect the transfer efficiency into cells, and therefore the said technique may become troublesome especially when many nucleic acids are involved, because every procedure requires the greatest care.

Under the conditions above, a method for transferring a nucleic acid into cells more conveniently and efficiently with less cytotoxicity is demanded to analyze functions of various genes exhaustively at cell level.

### DISCLOSURE OF INVENTION

The purpose of the present invention is to provide a novel method of nucleic acid transfer which is low-toxic and by which a nucleic acid can be transferred into cells conveniently and efficiently.

It was surprisingly found by the present inventors that a nucleic acid could be transferred into a cell efficiently and exert the function, just by mixing the nucleic acid (alone) and cells in a medium and then applying a high-concentration calcium chloride solution to the resultant mixture. This finding convinced the present inventors that calcium chloride and, further, a metal salt in general, could be useful as a nucleic acid transfer agent.

The present invention has been established on the basis of these findings.

The present invention encompasses the followings.
(1) A method of nucleic acid transfer comprising the following steps (a) and (b):
   (a) contacting a nucleic acid with a cell in a medium; and
   (b) following the step (a), contacting the medium of (a) with a high-concentration solution of a metal salt.
(2) The method of nucleic acid transfer according to (1) above, wherein the nucleic acid is a single-stranded DNA, a double-stranded DNA, a single-stranded RNA, a double-stranded RNA, an oligonucleotide or a ribozyme.
(3) The method of nucleic acid transfer according to (2) above, wherein the double-stranded DNA or the double-stranded RNA is in the linear or cyclic form.
(4) The method of nucleic acid transfer according to (3) above, wherein the cyclic double-stranded DNA is in the form of expression plasmid.
(5) The method of nucleic acid transfer according to (2) above, wherein the oligonucleotide is a deoxyribonucleotide, a ribonucleotide, phosphorothioate oligodeoxynucleotide, a 2'-O-(2-methoxy)ethyl-modified nucleic acid (2'-MOE-modified nucleic acid), a small interfering RNA (siRNA), a cross-linked nucleic acid (locked nucleic acid; LNA), a peptide nucleic acid (PNA) or a morpholino antisense nucleic acid.
(6) The method of nucleic acid transfer according to any one of (1) to (5) above, wherein the nucleic acid is in the form of a complex or an inclusion body with a biodegradable substance or a living body-derived substance.
(7) The method of nucleic acid transfer according to (6) above, wherein the living body-derived substance is atelocollagen.
(8) The method of nucleic acid transfer according to any one of (1) to (7) above, wherein the concentration of the high-concentration solution of a metal salt to be contacted with the medium obtained in the step (a) is within the range of 0.1 M - 3.0 M.
(9) The method of nucleic acid transfer according to (8) above, wherein the concentration of the high-concentration solution of a metal salt to be contacted with the medium obtained in the step (a) is within the range of 0.5M-2.0M.
(10) The method of nucleic acid transfer according to any one of (1) to (9) above, wherein the volume of the high-concentration solution of a metal salt to be contacted with the medium obtained in the step (a) is within the range of 1 µL - 20 µL per 500 µL of the medium of step (a).
(11) The method of nucleic acid transfer according to (10) above, wherein the volume of the high-concentration solution of a metal salt to be contacted with the medium obtained in the step (a) is within the range of 2 µL - 10 µL per 500 µL of the medium of step (a).
(12) The method of nucleic acid transfer according to any one of (1) to
(11) above, wherein the solution of a metal salt is a solution of a divalent metal chloride.
(13) The method of nucleic acid transfer according to (12) above, wherein the solution of a divalent metal chloride is a solution of calcium chloride.
(14) A nucleic acid transfer agent comprising a solid metal salt or a high-concentration solution of a metal salt as an ingredient.
(15) A nucleic acid transfer agent consisting of a solid metal salt or a high-concentration solution of a metal salt.
(16) The nucleic acid transfer agent according to (14) or (15) above, which is used in the method of nucleic acid transfer set forth in any one of (1) to (13) above.
(17) The nucleic acid transfer agent according to any one of (14) to (16) above, wherein the concentration of the high-concentration solution of a metal salt is within the range of 0.1 M - 6.0 M.
(18) The nucleic acid transfer agent according to (17) above, wherein the concentration of the high-concentration solution of a metal salt is within the range of 0.5 M - 4.0 M.
(19) The nucleic acid transfer agent according to any one of (14) to (18) above, wherein the metal salt is a chloride of divalent metal.
(20) The nucleic acid transfer agent according to (19) above, wherein the chloride of a divalent metal is calcium chloride.
(21) A kit for nucleic acid transfer which comprises a nucleic acid transfer agent set forth in any one of (14) to (20) above.
(22) Use of a nucleic acid transfer agent or a kit set forth in any one of (14) to (21) above in the nucleic acid transfer.
(23) A method of nucleic acid transfer, comprising the following steps (a) and (b):
   (a) contacting a nucleic acid with a cell in a medium; and
   (b) following the step (a), contacting a high-concentration solution
   of calcium chloride with the medium of (a).
(24) The method of nucleic acid transfer according to (23) above, wherein the nucleic acid is a single-stranded DNA, a double-stranded DNA, a single-stranded RNA, a double-stranded RNA, an oligonucleotide or a ribozyme.
(25) The method of nucleic acid transfer according to (24) above, wherein the double-stranded DNA or the double-stranded RNA is in the linear or cyclic form.
(26) The method of nucleic acid transfer according to (25) above, wherein the cyclic double-stranded DNA is in the form of expression plasmid.
(27) The method of nucleic acid transfer according to (24) above, wherein the oligonucleotide is a deoxyribonucleotide, a ribonucleotide, phosphorothioate oligodeoxynucleotide, a 2'-O-(2-methoxy)ethyl-modified nucleic acid (2'-MOE-modified nucleic acid), a small interfering RNA (siRNA), a cross-linked nucleic acid (locked nucleic acid; LNA), a peptide nucleic acid (PNA) or a morpholino antisense nucleic acid.
(28) The method of nucleic acid transfer according to any one of (23) to
(27) above, wherein the nucleic acid is in the form of a complex or an inclusion body with a biodegradable substance or a living body-derived substance.
(29) The method of nucleic acid transfer according to (28) above, wherein the living body-derived substance is atelocollagen.
(30) The method of nucleic acid transfer according to any one of (23) to
(29) above, wherein the concentration of the high-concentration solution of calcium chloride to be contacted with the medium obtained in the step (a) is within the range of 0.1 M - 3.0 M.
(31) The method of nucleic acid transfer according to (30) above, wherein the concentration of the high-concentration solution of calcium chloride to be contacted with the medium obtained in the step (a) is within the range of 0.5 M-2.0 M.
(32) The method of nucleic acid transfer according to any one of (23) to
(31) above, wherein the volume of the high-concentration solution of calcium chloride to be contacted with the medium obtained in the step (a) is within the range of 1 µL-20 µL per 500 µL of the medium of step (a).
(33) The method of nucleic acid transfer according to (32) above, wherein the volume of the high-concentration solution of calcium chloride to be contacted with the medium obtained in the step (a) is within the range of 2 µL-10 µL per 500 µL of the medium of step (a).
(34) A nucleic acid transfer agent comprising solid calcium chloride or a high-concentration solution of calcium chloride as an ingredient.
(35) A nucleic acid transfer agent consisting of solid calcium chloride or a high-concentration solution of calcium chloride.
(36) The nucleic acid transfer agent according to (34) or (35) above, which is used in the method of nucleic acid transfer set forth in any one of (23) to (33) above.
(37) The nucleic acid transfer agent according to any one of (34) to (36) above, wherein the concentration of the high-concentration solution of calcium chloride is within the range of 0.1 M - 6.0 M.
(38) The nucleic acid transfer agent according to (37) above, wherein the concentration of the high-concentration solution of calcium chloride is within the range of 0.5 M - 4.0 M.
(39) A kit for nucleic acid transfer which comprises a nucleic acid transfer agent set forth in any one of (34) to (38) above.
(40) Use of a nucleic acid transfer agent or a kit set forth in any one of (34) to (39) above in the nucleic acid transfer.

### BRIEF DESCRIPTION OF DRAWINGS

Figure 1 is a graph showing the results of an experiment wherein GFP expression plasmids were introduced into 293 cells by the method of gene transfer of the present invention.
Figure 2 is a graph showing the expression efficiency of GFP in 293 cells in an experiment wherein the cells were suspended in a medium to which calcium chloride was previously added.
Figure 3 is a graph showing the results of an experiment wherein GFP expression plasmids were introduced into HeLa cells by the gene transfer method of the present invention.
Figure 4 is a graph showing the results of an experiment wherein siRNAs were transferred into NEC8 cells by the gene transfer method of the present invention.
Figure 5 is a graph showing the results of an experiment wherein complexes of a GFP expression plasmid and atelocollagen were introduced into 293 cells and HeLa cells by the gene transfer method of the present invention.
Figure 6 is a graph showing the results of an experiment wherein complexes of a siRNA and atelocollagen were introduced into PC-3M-Luc-C6 cells by the gene transfer method of the present invention. A) shows the effects of siRNA transfer on human enhancer of zeste homolog 2 (EZH2), and B) shows the effects of siRNA transfer on phosphoinositide 3'-hydroxykinase p110-alpha subunit (p110-alpha).

### BEST MODE FOR CARRYING OUT THE INVENTION

The present invention provides a method of gene transfer comprising at least the following steps (a) and (b):
(a) contacting a nucleic acid with a cell in a medium; and
(b) following the step (a), contacting the medium of (a) with a high-concentration solution of a metal salt.

In light of the principle underlying the method of gene transfer of the present invention, any kinds of nucleic acids can be used as the objective nucleic acid to be transferred without limitation. In other words, the nucleic acid may be any of polynucleotides (DNA, RNA), oligonucleotides, ribozymes, and the like, and can take any forms of a single-stranded, double-stranded or analogues thereof. Specifically, examples of nucleic acid of the present invention include a single-stranded DNA, a double-stranded DNA, a single-stranded RNA, a double-stranded RNA, an oligonucleotide and a ribozyme.

When the nucleic acid of the present invention is a double-stranded DNA or a double-stranded RNA, it may be in the linear or cyclic form. Further, when the nucleic acid of the present invention is a cyclic double-stranded DNA, it may be in the form of plasmid. The said plasmid may be an expression plasmid or a non-expression plasmid.

When the nucleic acid of the present invention is a single-stranded DNA or a single-stranded RNA, the both of sense-strand and anti-sense-strand can be used.

When the nucleic acid of the present invention is an oligonucleotide, there are no limitations on the kinds of oligonucleotide to be transferred, and a single-stranded oligonucleotide, a double-stranded oligonucleotide, or an analogue thereof can be used. Specifically, examples include deoxyribonucleotide (DNA), ribonucleotide (RNA), phosphorothioate oligodeoxynucleotide, 2'-O-(2-methoxy)ethyl-modified nucleic acid (2'-MOE-modified nucleic acid), small interfering RNA (siRNA), cross-linked nucleic acid (locked nucleic acid: LNA ; Singh, et al, *Chem.Commun.,* 455, 1998), peptide nucleic acid (Peptide Nucleic Acid: PNA; Nielsen, et al., *Science,* 254, 1497, 1991), and morpholino antisense nucleic acid (Summerton and Weller, *Antisense & Nucleic Acid Drug Development,* 7, 187, 1997).

The nucleic acids described above can be used at a concentration used in the conventional gene transfer ranging from 0.001 to 1,000 µg/mL.

The nucleic acids described above can be used as a solution in a solvent that does not disturb the cell culture. Examples of such a solvent include distilled water, physiological saline, HEPES buffer (Sigma), TRIS buffer (Sigma), PBS buffer (Invitrogen), cell culture medium, and the like.

The above-mentioned nucleic acid may be in the form of a complex with or an inclusion body in a biodegradable substance or a living body-derived substance which substance is not cytotoxic. Examples of biodegradable substance include polylactic acid, polyglycolic acid, and a copolymer thereof, a lactone polymer, a polyethylene glycol polymer, and the like. Examples of a living body-derived substance include chitosan, gelatine, collagen, enzyme-solubilized collagen (atelocollagen), and modified derivatives thereof. A complex or an inclusion body of a nucleic acid with a biodegradable substance or a living body-derived substance can be prepared according to the teaching in a literature, such as Panyman et al, Biodegradable nanoparticles for drug and gene delivery to cells and tissue., *Adv Drug Deliv Rev.* 2003; 55(3):329-47; Li XW et al, Sustained expression in mammalian cells with DNA complexed with chitosan nanoparticles., *Biochem Biophys Acta.* 2003; 1630(1):7-18; or WO 03/000297.

Preferred living body-derived substances are collagen and enzyme-solubilized collagen (atelocollagen) of any kinds, origins, and types. For example, substances of unmodified or modified-type can be illustrated. Examples of modified substance usable include those obtained through the chemical modification or chemical and/or physical crosslinking at the side-chain amino- or carboxyl-group.

The collagen can be used as a solution of a concentration ranging from 0.00001% to 3% (0.0001 mg/mL - 30 mg/mL), preferably from 0.0001% to 0.3%, more preferably from 0.0005 % to 0.1%.

A nucleic acid can be stabilized and released in a sustained manner when it forms a complex or an inclusion body with the above-described biodegradable substance or the living body-derived substance. Accordingly, it is possible to sustain the effects of nucleic acid by introducing the resultant complex or the inclusion body into cells.

The cells to which the method of nucleic acid transfer of the present invention is applied are not limited to any particular cells in light of the principle underlying the present method. Specifically, the method of nucleic acid transfer of the present invention can be applied to fibrocytes, epithelial cells, endothelial cells, neuroblasts, lymphoblasts, floating cells, astrocytes, round cells, spindle cells, ameboid cells, and the like.

Any medium can be used in the method of nucleic acid transfer of the present invention as long as it neither causes death to cells nor disturbs the nucleic acid incorporation by cells according to the method of the present invention. Specifically, examples include a culture medium, a buffer, or a culture medium or a buffer containing serum, which is used conventionally in the cell culture.

The culture medium can be any medium as long as it is suited to each cell. Examples of medium include RPMI1640 (Invitrogen), DULBECCO'S MODIFIED EAGLE MEDIA (Invitrogen), F-10 Nutrient Mixture (Invitrogen), F-12 Nutrient Mixture (Invitrogen), Iscove's Modified Dulbecco's Media (Invitrogen), MINIMUM ESSENTIAL MEDIA (Invitrogen), and the like.

Examples of a buffer include HEPES buffer (Sigma), TRIS buffer (Sigma), PBS buffer (Invitrogen), and the like.

Examples of serum include fetal bovine serum, bovine serum, calf serum, horse serum, and the like. There are no limitations on the concentration of serum in a medium as long as it is suited for cell culture. The concentration may be preferably 0 - 20% (v/v), and more preferably 5 - 10% (v/v).

Any metal salt solution can be used as the "metal salt solution" in the method of nucleic acid transfer of the present invention within the limits that the cell culture is not influenced. Whether or not a metal salt solution possibly exerts influence on the cell culture can be easily tested by comparing the cell-growth rate (cell density) or the like, in a cell culture containing the metal salt solution and that in a cell culture free from the said metal salt solution.

Specifically, examples of a metal salt include salts of a metal such as calcium, potassium, magnesium, sodium, manganese, iron, copper, zinc, and the like. More specifically, examples of said metal salt include hydrochlorides, phosphates, sulfates, carbonates or nitrates of the above-mentioned metals. Hydrochlorides of the above-mentioned metal are preferred, and chlorides of a divalent metal are more preferred.

Specifically, examples of a chloride of a divalent metal include calcium chloride, magnesium chloride, zinc chloride, ferrous chloride, manganese chloride, and the like, and calcium chloride is preferred. Thus, the most preferred metal salt solution of the present invention is calcium chloride solution.

The metal salt solution of the present invention may contain as an ingredient(s) either a single metal salt or a combination of two or more metal salts as mentioned above.

A metal salt solution (preferably, calcium chloride solution) used in the method of nucleic acid transfer of the present invention is a high-concentration metal salt solution. The term "high-concentration" used herein refers to a concentration of 0.1 M or greater, specifically, to a concentration within the range of 0.1 M - 3.0 M, preferably 0.3 M - 3.0 M, more preferably 0.5 M - 3.0 M, further preferably 0.5 M - 2.5 M, especially preferably 0.5 M - 2.0 M, and most preferably 1.0 M - 2.0 M.

Any solvent can be used to dissolve the above-mentioned metal salt as long as it does not disturb the cell culture. Examples of such a solvent include distilled water, physiological saline, HEPES buffer (Sigma), TRIS buffer (Sigma), PBS buffer (Invitrogen), cell culture medium, and the like.

The method of nucleic acid transfer of the present invention will be hereinafter described in detail.

First, a nucleic acid to be transferred is contacted with a cell as the introduction target in a medium. The contact is conducted in a culture vessel suited for the ordinary cell culture. Examples of a culture vessel include a dish, a flask, a multiple-well plate and the like for cell culture.

Examples of the method whereby to contact a nucleic acid with a cell include: a method wherein nucleic acids are added to a cell suspension medium followed by plating onto a cell culture vessel; a method wherein cells are suspended in a medium to which nucleic acids are previously added, followed by plating onto a cell culture vessel; a method wherein cells are suspended into a culture medium and plated onto a cell culture vessel, followed by addition of nucleic acids; a method wherein nucleic acids are first added to a cell culture vessel, and a culture medium into which cells are previously suspended is added thereto; and a method wherein an aqueous nucleic acid solution is added to a cell culture vessel and made dry or adsorb to the vessel, and a culture medium into which cells are previously suspended is added thereto.

The nucleic acid used herein may be in the form of a complex or an inclusion body with a biodegradable substance or a living body-derived substance, as mentioned above. Particularly, such a complex or an inclusion body can be conveniently used in the method wherein an aqueous nucleic acid solution is added to a cell culture vessel and made dry or adsorb to the vessel.

A particular example includes a method wherein a solution of a complex of a nucleic acid solution with an aqueous atelocollagen solution is added to a multiple-well plate and made dry, and thereto is added a culture medium to which cells have been suspended.

There are no particular limitations on the amount (concentration) of nucleic acids and the cell number (density) to be contacted, as long as they are within the range that are generally used in the gene transfer. Further, temperature at the time of contact may be within the range from 0°C to 42°C, preferably, from room temperature to 37°C.

Next, a high-concentration solution of a metal salt (preferably, a high-concentration solution of calcium chloride) is contacted with the medium above in which nucleic acids have been contacted with cells (hereinafter, said medium may be referred to as "medium of step (a)"). For example, it can be carried out by adding a high-concentration solution of a metal salt to a culture vessel containing the medium of step (a), or by adding the medium of step (a) to a cell culture vessel to which a high-concentration solution of a metal salt is previously added.

The timing of contacting (adding) a high-concentration solution of a metal salt is not limited particularly; however, it is appropriate that the high-concentration solution of a metal salt is allowed to contact with cells within 2 hours, preferably within 30 minutes, more preferably within 10 minutes after contacting the cells with nucleic acids.

The amount of a high-concentration solution of a metal salt (preferably, a high-concentration solution of calcium chloride) to be contacted (added) is not limited particularly as long as the nucleic acids are transferred into cells satisfactory; however, it is preferred that from 1 µL to 20 µL of a high-concentration solution of a metal salt is contacted with (added to) 500 µL of the medium of step (a). More preferably, from 2 µL to 10 µL, still more preferably, from 5 µL to 10 µL of a high-concentration solution of a metal salt is contacted with (added to) 500 µL of the medium of step (a).

More specifically, since a 24-well plate, for example, generally contains about 500 µL of the medium of step (a) per well, it is appropriate that a high-concentration solution of a metal salt is added at 1 µL - 20 µL/well, preferably 2 µL - 10 µL/well, more preferably 5 µL - 10 µL/well.

After adding a high-concentration solution of a metal salt, the culture vessel is stirred to mix the metal salt uniformly with the medium of step (a), and cultivation is conducted for about 1 hour to 1 day. The condition for cultivation is not limited particularly as long as it does not adversely affect the nucleic acid transfer into cells. However, cultivation can be carried out in the presence of 5% CO₂ and at a temperature of from 0°C to 42°C, preferably from room temperature to 37°C, and more preferably at 37°C.

The nucleic acid transfer can be established according to the above-mentioned procedures.

The method of nucleic acid transfer of the present invention as described above can be applied not only to the functional analysis of a gene at cell level, but also the production of genetically engineered cell lines, and the nucleic acid transfer into cells in the ex vivo gene therapy.

The present invention provides a nucleic acid transfer agent, which is used in the method of nucleic acid transfer of the present invention.

The nucleic acid transfer agent of the present invention is characterized in that it comprises a solid metal salt or a high-concentration solution of a metal salt as an ingredient. Specifically, examples include a nucleic acid transfer agent consisting of a solid metal salt or a high-concentration solution of a metal salt.

As the "metal salt", any metal salt solution can be used within the limits that the cell culture is not influenced. Whether or not a metal salt solution possibly exerts influence on the cell culture can be easily tested by comparing the cell-growth rate (cell density) or the like, in a cell culture containing the metal salt solution and that in a cell culture free from the said metal salt solution.

Specifically, examples of a metal salt include salts of a metal such as calcium, potassium, magnesium, sodium, manganese, iron, copper, zinc, and the like. More specifically, examples of said metal salt include hydrochlorides, phosphates, sulfates, carbonates or nitrates of the above-mentioned metals. Hydrochlorides of the above-mentioned metal are preferred, and chlorides of a divalent metal are more preferred.

Specifically, examples of a chloride of a divalent metal include calcium chloride, magnesium chloride, zinc chloride, ferrous chloride, manganese chloride, and the like, and calcium chloride is preferred. Thus, as a preferred embodiment of nucleic acid transfer agent of the present invention, the present invention provides a nucleic acid transfer agent comprising solid calcium chloride or a high-concentration solution of calcium chloride as an ingredient. More specifically, the present invention provides a nucleic acid transfer agent consisting of solid calcium chloride or a high-concentration solution of calcium chloride.

The nucleic acid transfer agent of the present invention may contain as an ingredient(s) either a single metal salt or a combination of two or more metal salts mentioned above.

When the nucleic acid transfer agent of the present invention contains a high-concentration solution of a metal salt as an ingredient, the concentration may be equal to or greater than 0.1 M.

As mentioned above, the concentration of a metal salt solution that is contacted with a medium of step (a) can be within the range of 0.1 M - 3.0 M, preferably 0.3 M - 3.0 M, more preferably 0.5 M - 3.0 M, further preferably 0.5 M - 2.5 M, especially preferably 0.5 M - 2.0 M, and most preferably 1.0 M - 2.0 M. Accordingly, the concentration of a metal salt in the nucleic acid transfer agent of the present invention should be adjusted so that it gives the above-mentioned concentration when used after dilution or by itself.

Thus, when the nucleic acid transfer agent of the present invention comprises a high-concentration solution of a metal salt (preferably, a high-concentration solution of calcium chloride) as an ingredient, the concentration may be 0.1 M or greater, preferably 0.1 M - 6.0 M, more preferably 0.1 M - 4.0 M, still more preferably 0.5 M - 4.0 M.

Any solvent can be used to dissolve the above-mentioned metal salt as long as it does not disturb the cell culture. Examples of such a solvent include distilled water, physiological saline, HEPES buffer (Sigma), TRIS buffer (Sigma), PBS buffer (Invitrogen), cell culture medium, and the like.

The above-mentioned nucleic acid transfer agent of the present invention can be a component of a kit for nucleic acid transfer. The kit may contain only the nucleic acid transfer agent of the present invention or a combination of the nucleic acid transfer agent of the present invention and other component(s). Examples of other components of the kit include fluorescently labeled oligonucleotides, positive control siRNAs, and the like. When the kit contains a solid metal salt as a component, it may further contain a solvent for dissolving the same such as distilled water, physiological saline, HEPES buffer (Sigma),TRIS buffer (Sigma), PBS buffer (Invitrogen), cell culture medium, or the like.

### EXAMPLES

The present invention is further illustrated by the following examples, but should not be construed as being limited thereto.

### Example 1

### Study on the Method of Gene Transfer (1)

An aqueous solution of 100 µg/mL GFP expression plasmid (100 µL) was added to each well of a 24-well cell culture plate, and dried by blowing cool air. Human adrenogenic epithelial cell line, 293 cells (ATCC: Cell Biology Collection) were suspended into the DMEM medium (Sigma) containing 10% fetal bovine serum (FBS), and seeded at 2.5 x 10⁴ cells (500 µL)/well. Immediately after the cells were seeded, an aqueous solution of 1.7 M calcium chloride (0, 1.5, 2.5, 3.5, 5.0, 6.5, or 8.0 µL) was added and the plate was stirred to mix uniformly. The final concentration of calcium chloride in each medium was 1.8 mM, 7.1 mM, 10.2 mM, 14.2 mM, 19.5 mM, 24.8 mM, or 30.1 mM. On day 2 after seeding, cells were observed with fluorescence microscope, and the cells expressing GFP were counted to calculate the transfer efficiency.

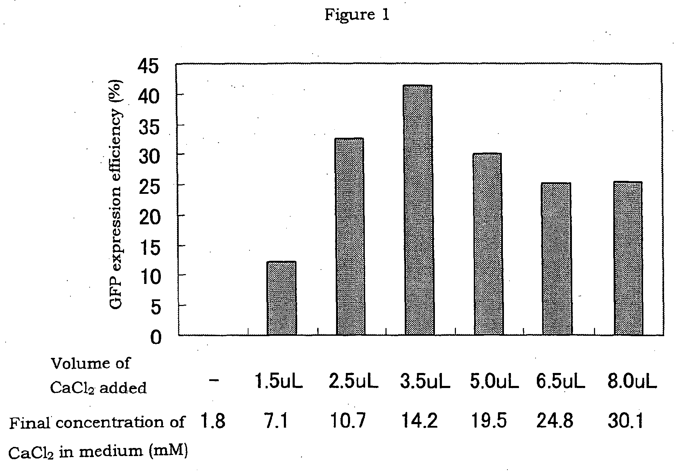

The results are shown in Fig. 1. As is clear from the Fig. 1, genes could be introduced efficiently by adding a high-concentration (1.7 M) calcium chloride solution. Neither morphological changes nor death of cells was observed. In the 24-well plate used, excellent gene expression efficiency was observed when 1.7 M calcium chloride solution was added to each well at a volume ranging from 2.5 to 8.0 µL.

### Example 2

### Study on the Method of Nucleic Acid Transfer (2)

The gene transfer efficiency was examined in the same manner as Example 1 except that cells were suspended into a medium to which calcium chloride solution was previously added before seeding into a well. An aqueous solution of 100 µg/mL GFP expression plasmid (100 µL) was added to each well of a 24-well cell culture plate, and dried by blowing cool air. A calcium chloride solution was added to a medium to obtain media each containing calcium chloride at a concentration of 1.8 mM, 7.1 mM, 10.2 mM, 14.2 mM, 19.5 mM, 24.8 mM or 30.1 mM. Into the respective media were suspended 293 cells, and seeded at 2.5 x 10⁴ cells (500 µL)/well. On day 2 after seeding, cells were observed with fluorescence microscope. Cells expressing GFP were counted to calculate the transfer efficiency.

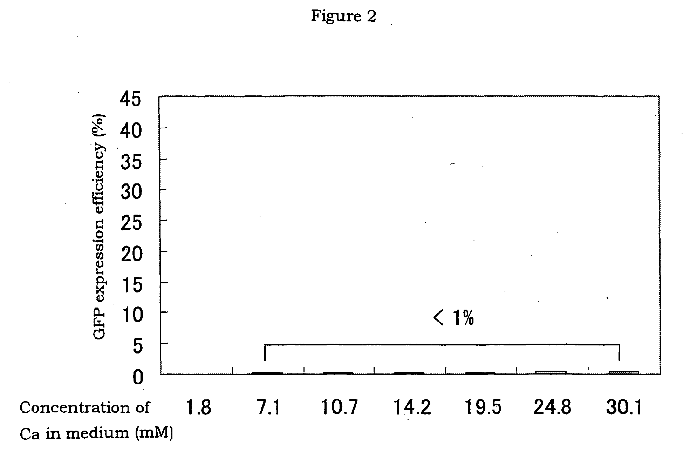

The results are shown in Fig. 2. In contrast to the results obtained in Example 1 (Fig. 1), genes could not be introduced when cells were previously suspended into a medium containing calcium chloride solution before seeding onto the plate. The results obtained in the Examples 1 and 2 indicated that the facilitatory effect on gene transfer into cells is not resulted from the increase of calcium chloride solution in a medium but the manner of adding the calcium chloride solution. It was revealed to be important that cells are previously mixed with genes in advance to contact with a high-concentration solution of calcium chloride.

### Example 3

### Gene Transfer into HeLa Cells by the Method of Nucleic Acid Transfer of the Present Invention

It was examined whether the method of gene transfer of the present invention is applicable to cells different from those used in Example 1.

An aqueous solution of 100 µg/mL GFP expression plasmid (100 µL) was added to a 24-well cell culture plate, and dried by blowing cool air. Human cervical cancer-derived epithelial cell line, HeLa cells (ATCC: Cell Biology Collection) were suspended into the DMEM medium (Sigma) containing 10% FBS, and seeded at 1.5 x 10⁴ cells (500 µL)/well. Immediately after the cells were seeded, an aqueous solution of 1.7 M calcium chloride (0, 1.5, 2.5, 3.5, 5.0, 6.5, or 8.0 µL) was added and the plate was stirred to mix uniformly. On day 4 after seeding, cells were observed with fluorescence microscope, and the cells expressing GFP were counted to calculate the transfer efficiency.

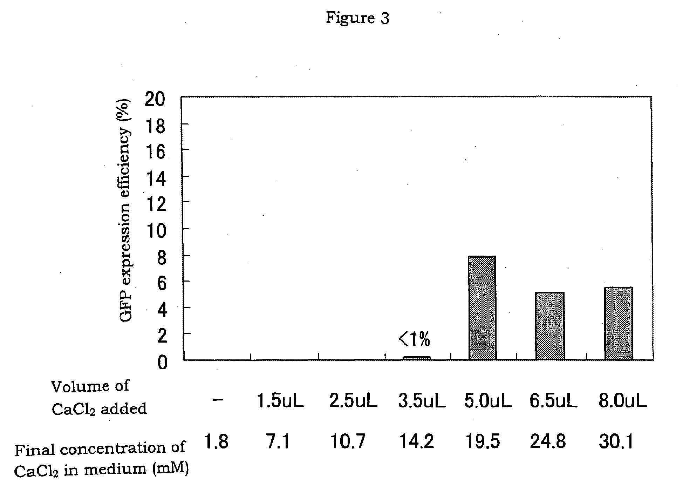

The results are shown in Fig. 3. Genes could be introduced efficiently into HeLa cells by adding 1.7 M calcium chloride solution after cells were seeded. Neither morphological changes nor death of cells was observed. These results demonstrated that the method of the present invention does not depend on the kinds of cells.

### Example 4

### Transfer of siRNA by the Method of Nucleic Acid Transfer of the Present Invention

It was examined whether a nucleic acid other than an expression plasmid can be introduced by the method of gene transfer of the present invention.

As a nucleic acid to be introduced, siRNA (hereinafter, referred to as "hEx3-1") which specifically inhibits human FGF-4 mRNA was used. As a cell into which a nucleic acid is introduced, human testicular tumor-derived epithelial cell line, NEC8 (ATCC: Cell Biology Collection) expressing intensely human FGF-4 protein was used.

An aqueous solution of 10 µg/mL hEx3-1 (350 µL) was added to a 6-well cell culture plate, and dried by blowing cool air. NEC8 cells were suspended into the DMEM medium (Sigma) containing 10% FBS and seeded at 3.75 x 10⁵ cells (1.5 mL)/plate. Immediately after the cells were seeded, an aqueous solution of 1.7 M calcium chloride (20 µL) was added and the plate was stirred to mix uniformly. In control, a well(s) not treated with hEx3-1 for coating followed by drying were subjected to the same procedures. On day 3 after seeding, media were recovered and the concentration of FGF-4 in each medium was determined by ELISA (Human FGF-4 Quantikine ELISA kit; R&D Systems). Further, cells were separated from the medium and the protein content was determined by Bradford method (Bio-Rad Protein Assay; BioRad). The concentration of FGF-4 in the medium was divided by the amount of protein to calculate the yield of FGF-4 in respective wells.

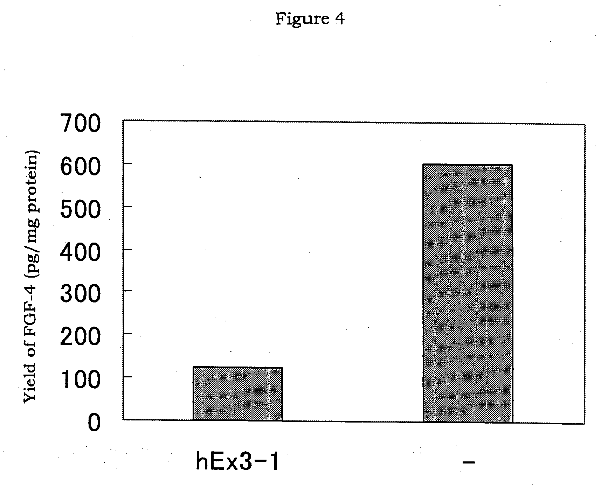

The results are shown in Fig. 4. Introduction of hEx3-1 resulted in the inhibition of FGF-4 production into medium. Thus, it was demonstrated that siRNA was transferred into NEC8 cells efficiently and expressed the intended activity satisfactory. These results indicated that the method of nucleic acid transfer of the present invention does not depend on the kinds of nucleic acids.

### Example 5

### Introduction of a Complex of a Gene and a Living Body-derived Substance by the Method of Nucleic Acid Transfer of the Present Invention

It was examined whether a complex of a gene and a living body-derived substance which has effects of stabilizing and sustaining the release of a gene can be introduced by the method of gene transfer of the present invention.

As a living body-derived substance, atelocollagen (Koken, Inc.) was used.

A solution containing complexes was prepared by mixing equal volumes of an aqueous solution of GFP expression plasmid (200 µg/mL) and an aqueous solution of 0.016 % atelocollagen. The complex solution (100 µL) was added to each well of a 24-well cell culture plate, and dried by blowing cool air. Human adrenogenic epithelial cell line, 293 cells or human cervical cancer-derived epithelial cell line, HeLa cells (ATCC: Cell Biology Collection) were suspended into the DMEM medium (Sigma) containing 10% fatal bovine serum (FBS), and seeded at 2.5 x 10⁴ cells (500 µL)/well. Immediately after the cells were seeded, an aqueous solution of 1.7 M calcium chloride (5.0 µL) was added and the plate was stirred to mix uniformly. On day 2 after seeding, cells were observed with fluorescence microscope, and the cells expressing GFP were counted to calculate the transfer efficiency.

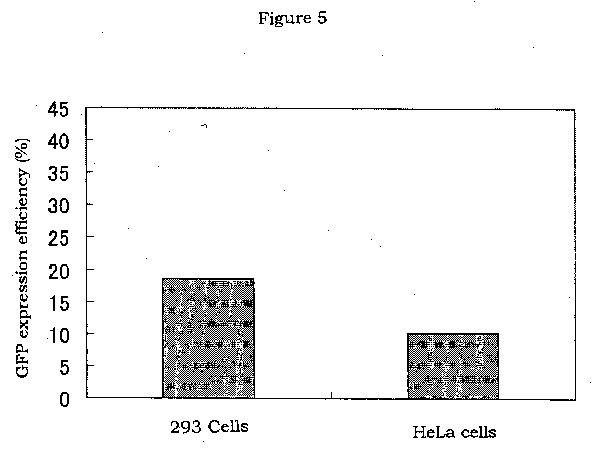

The results are shown in Fig. 5. As is clear from the Fig. 5, complexes of atelocollagen could be introduced into cells by adding 1.7 M calcium chloride solution into a well after cells were seeded. These results demonstrated that it is possible to make a nucleic acid exert sustained effects by transferring the nucleic acid as a complex.

### Example 6

### Introduction of a Complex of a siRNA and a Living Body-derived Substance by the Method of Nucleic Acid Transfer of the Present Invention

A solution containing complexes was prepared by mixing equal volumes of an aqueous solution of 0.016 % atelocollagen and an aqueous solution of 300 nM small interfering RNA (siRNA) for either the human enhancer of zeste homolog 2 (EZH2) or the phosphoinositide 3'-hydroxykinase p110-alpha subunit (p110-alpha). The complex solution (250 µL) was added to each well of a 6-well cell culture plate, and dried by blowing cool air. Human prostate cancer derived cell line, PC-3M-Luc-C6 cells (Xenogen Corp.) were seeded at 5 x 10⁴ cells/well. Immediately after the cells were seeded, an aqueous solution of 1.7 M calcium chloride (20 µL) was added. On day 4 after the cells were seeded, RNA was extracted and cDNA was synthesized. Expression amount of mRNA was analyzed by quantitative PCR assay. The results were corrected on the basis of expression amount of GAPDH used as the internal standard.

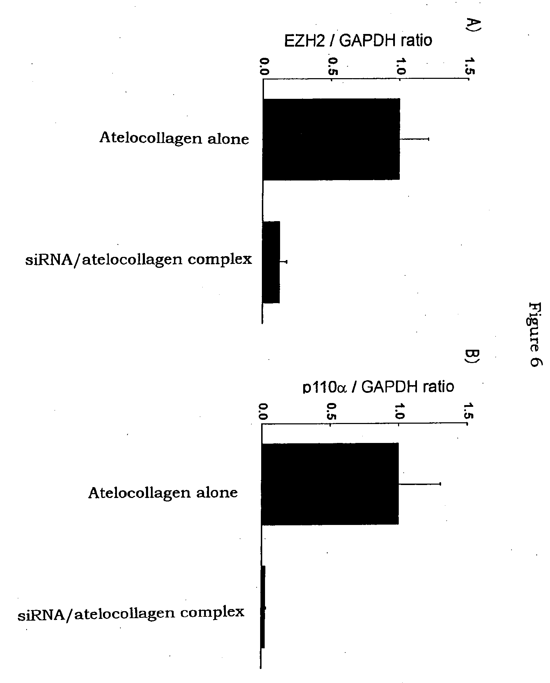

The results are shown in Fig. 6. As is clear from the Fig. 6, siRNA could be transferred into cells by adding 1.7 M calcium chloride solution to a well after cells were seeded.

### INDUSTRIAL APPLICABILITY

According to the present invention, it is provided a novel method of nucleic acid transfer. The method of nucleic acid transfer of the present invention is convenient and less cytotoxic, and shows high transfer efficiency, and also is low-cost. It can be used extensively without distinction of kinds of cells or nucleic acids.

## Claims

1. A method of nucleic acid transfer comprising the following steps (a) and (b):
(a) contacting a nucleic acid with a cell in a medium; and
(b) following the step (a), contacting the medium of (a) with a high-concentration solution of a metal salt.

2. The method of nucleic acid transfer according to claim 1, wherein the nucleic acid is a single-stranded DNA, a double-stranded DNA, a single-stranded RNA, a double-stranded RNA, an oligonucleotide or a ribozyme.

3. The method of nucleic acid transfer according to claim 2, wherein the double-stranded DNA or the double-stranded RNA is in the linear or cyclic form.

4. The method of nucleic acid transfer according to claim 3, wherein the cyclic double-stranded DNA is in the form of expression plasmid.

5. The method of nucleic acid transfer according to claim 2, wherein the oligonucleotide is a deoxyribonucleotide, a ribonucleotide, a phosphorothioate oligodeoxynucleotide, a 2'-O-(2-methoxy)ethyl-modified nucleic acid (2'-MOE-modified nucleic acid), a small interfering RNA (siRNA), a cross-linked nucleic acid (locked nucleic acid; LNA), a peptide nucleic acid (PNA) or a morpholino antisense nucleic acid.

6. The method of nucleic acid transfer according to any one of claims 1 to 5, wherein the nucleic acid is in the form of a complex or an inclusion body with a biodegradable substance or a living body-derived substance.

7. The method of nucleic acid transfer according to claim 6, wherein the living body-derived substance is atelocollagen.

8. The method of nucleic acid transfer according to any one of claims 1 to 7, wherein the concentration of the high-concentration solution of a metal salt to be contacted with the medium obtained in the step (a) is within the range of 0.1 M - 3.0 M.

9. The method of nucleic acid transfer according to claim 8, wherein the concentration of the high-concentration solution of a metal salt to be contacted with the medium obtained in the step (a) is within the range of 0.5 M - 2.0 M.

10. The method of nucleic acid transfer according to any one of claims 1 to 9, wherein the volume of the high-concentration solution of a metal salt to be contacted with the medium obtained in the step (a) is within the range of 1 µL - 20 µL per 500 µL of the medium of step (a).

11. The method of nucleic acid transfer according to claim 10, wherein the volume of the high-concentration solution of a metal salt to be contacted with the medium obtained in the step (a) is within the range of 2 µL - 10 µL per 500 µL of the medium of step (a).

12. The method of nucleic acid transfer according to any one of claims 1 to 11, wherein the solution of a metal salt is a solution of a divalent metal chloride.

13. The method of nucleic acid transfer according to claim 12, wherein the solution of a divalent metal chloride is a solution of calcium chloride.

14. A nucleic acid transfer agent comprising a solid metal salt or a high-concentration solution of a metal salt as an ingredient.

15. The nucleic acid transfer agent according to claim 14, which is used in the method of nucleic acid transfer set forth in any one of claims 1 to 13.

16. The nucleic acid transfer agent according to claim 14 or 15, wherein the concentration of the high-concentration solution of a metal salt is within the range of 0.1 M - 6.0 M.

17. The nucleic acid transfer agent according to claim 16, wherein the concentration of the high-concentration solution of a metal salt is within the range of 0.5 M - 4.0 M.

18. The nucleic acid transfer agent according to any one of claims 14 to 17, wherein the metal salt is a chloride of divalent metal.

19. The nucleic acid transfer agent according to 18, wherein the chloride of a divalent metal is calcium chloride.

20. A kit for nucleic acid transfer which comprises a nucleic acid transfer agent set forth in any one of claims 14 to 19.

21. Use of a nucleic acid transfer agent or a kit set forth in any one of claims 14 to 20 in the nucleic acid transfer.
